# EUROPEAN PATENT SPECIFICATION

(11) **EP 1 861 039 B1**
(45) Date of publication and mention of the grant of the patent: **10.03.2010**
(21) Application number: 06717738.6
(22) Date of filing: 09.01.2006
(51) Int. Cl.: A61F 2/06

(54) **COATINGS FOR USE ON MEDICAL DEVICES**
BESCHICHTUNGEN ZUR VERWENDUNG AUF MEDIZINPRODUKTEN
REVÊTEMENTS POUR UTILISATION SUR DES APPAREILS MÉDICAUX

(30) Priority: 21.03.2005 US 85780
(43) Date of publication of application: 05.12.2007
(73) Proprietor: Boston Scientific Limited, Hastings Christ Church (BB)
(72) Inventor: WEBER, Jan, 6228 GJ Maastricht (NL); JAGGER, Karl, Deephaven, Minnesota 55331 (US); ATANASOSKA, Liliana, Edina, Minnesota 55436 (US)
(74) Representative: Altenburg, Udo
(86) International application number: PCT/US2006/000574
(87) International publication number: WO 2006/101573

(56) References cited:
- EP-A- 0 923 953
- WO-A-98/20812
- WO-A-2006/026201
- US-A- 5 100 429
- US-A1- 2002 013 599
- US-B1- 6 506 202

## Description

### FIELD OF THE INVENTION

The present invention relates to the field of delivery systems for medical devices, in particular, to expandable members employed for the delivery of stents, and to coatings employed thereon.

### BACKGROUND OF THE INVENTION

Medical device such as stents and stent delivery assemblies are utilized in a number of medical procedures, and as such their structure and function are well known. A stent is a generally cylindrical radially expandable prosthesis introduced percutaneously via a catheter into a lumen of a body vessel in a configuration having a generally reduced diameter and then expanded to the diameter of the vessel. In its expanded configuration, the stent supports and reinforces the vessel walls while maintaining the vessel in an open, unobstructed condition.

Stents may be implanted in a variety of body lumens or vessels such as within the vascular, urethral, ureteral, reproductive, biliary, neurological, tracheal, cerebral, gastrointestinal, esophageal systems, etc.

Both self-expanding and inflation expandable stents are well-known and widely available. Self-expanding stents are typically maintained under positive external pressure in order to maintain their reduced diameter configuration during delivery of the stent to its deployment site. Inflation expandable stents are generally crimped to their reduced diameter about an expandable member of a delivery device, positioned at the deployment site, and expanded via outward radial pressure such as provided during inflation of the expandable member.

During a medical procedure, the stent is positioned in a precise location within a bodily lumen. To facilitate the proper positioning of a stent, it is desirable to prevent any unwanted relative movement between any of the stent, the balloon, the catheter and the interior of the vessel. This goal is rendered more difficult because the trend in stent design is to utilize thinner and more flexible structures which provide less radial inward force in the crimped state, hence there is less securement between the balloon and the stent. Slippage may occur during insertion of the stent through a guide catheter, while crossing tortuous anatomy, or during deployment of the stent.

The issue of slippage of a stent relative to a balloon has been dealt with in several different ways including by varying the coefficient of friction of the exposed portion of a balloon between the uninflated and inflated states of the balloon. Another approach involves providing a balloon with enlarged ends and a middle section of reduced diameter to retain a stent. Other approaches are non-balloon based, providing stent retention devices that extend from the catheter and engage the stent.

It is known to fabricate multi-layer films using the concept of electrostatic interaction between oppositely charged species during a stepwise absorption from an aqueous solution. Such multi-layer films have been employed in making capsules and in the development of functional colloidal particles.

US-A-5 100 429 and US-B-6 506 202 discloses, respectively, a catheter assembly having an adhesive coating for securing and releasing a stent mounted on a balloon.

### SUMMARY OF THE INVENTION

It is a goal of the present invention to provide a medical device delivery system using novel coating technology to improve medical device deployment accuracy by preventing slippage of the medical device during delivery of the device to the desired bodily location and during deployment of the device so as to facilitate the positioning of a medical device with greater precision.

This goal is achieved according to the invention by a catheter assembly as defined in claim 1. Advantageous embodiments of the invention are defined in the dependent claims.

In one aspect, the present invention relates to a novel coating for use on medical device components.

In one aspect, the novel coating is employed on components of catheter assemblies.

In one aspect, the novel coating is employed on an expandable medical balloon.

In another aspect, the expandable medical balloon may be disposed on the distal end of a catheter delivery assembly and used for securement of an intraluminal medical device during delivery to a deployment site within a patient's body lumen. The novel coatings according to the invention are disposed on at least a portion of the expandable medical balloon, the intraluminal medical device, or both.

In another aspect, a self-expanding intraluminal medical device is disposed about an inner member of a catheter delivery assembly, a degradable coating according to the invention is provided for securement of the self-expanding intraluminal medical device to the inner member.

The novel coating is suitably biocompatible, may be rapidly degrading or dissolving, and is applied as a thin layer to the medical device components.

In one aspect, the coating is a layer-by-layer (LbL) coating having at least one first layer and one second layer, the first layer including a positively charged material, and the second layer adjacent the first layer including a negatively charged material.

Alternatively, the first layer may include a negatively charged material and the second layer may include a positively charged material as well.

In any of the embodiments described herein, a therapeutic agent or mixtures of therapeutic agents may be optionally employed.

Furthermore, the present invention can be employed in combination with a drug eluting coating layer.

In one embodiment, the degradable coating is employed as an intermediate layer between a medical balloon and a stent having a drug eluting coating layer.

The coating is sufficiently strong to secure an intraluminal medical device during delivery to deployment sites within a patient's vasculature, but yet allows the intraluminal medical device to expand and release from an expandable balloon member once the expandable balloon member has been deflated.

These and other aspects, embodiments and advantages of the present invention will become immediately apparent to those of ordinary skill in the art upon review of the Detailed Description and Claims to follow.

### BRIEF DESCRIPTION OF THE DRAWINGS

FIG. 1 is a longitudinal cross-sectional side view of a catheter assembly having a balloon of the present invention mounted thereon and a stent disposed on the balloon.
FIG. 2 is an enlarged view taken at section 2 in FIG. 1.
FIG. 3 is a longitudinal side view of a stent disposed on a medical balloon representing not an embodiment of the present invention.
FIG. 4 is a longitudinal side view of a stent disposed on a medical balloon and having a coating disposed over the stent and balloon representing not an embodiment of the present invention.
FIG. 5 is a longitudinal side view of a stent and medical balloon similar to that shown in FIG. 4 with the balloon inflated and the stent in an expanded form representing not an embodiment of the present invention.
FIG. 6 is a longitudinal side view of a stent and balloon similar to that shown in FIG. 5 with the stent expanded and the balloon contracted and shown within a body vessel.
FIG. 7 is a fragmentary cross-section of a stent and balloon taken along the longitudinal axis of the balloon and having a layer-by-layer coating disposed between according to the invention.
FIG. 8 is a fragmentary cross-section of a stent and balloon similar to that shown in FIG. 7 taken along the longitudinal axis of the balloon with the stent shown in contact with a body vessel.
FIG. 9 is a fragmentary cross-section of a stent and balloon similar to that shown in FIG. 8 taken along the longitudinal axis of the balloon with the stent in an expanded state and the balloon in a contracted state.
FIG. 10 is a fragmentary cross-section of a stent and balloon taken along the longitudinal axis of the balloon and having an alternative embodiment of a layer-by-layer coating according to the invention.
FIG. 11 is a fragmentary cross-section of a stent and balloon similar to that shown in FIG. 10 taken along the longitudinal axis of the balloon, the stent crimped on the balloon.
FIG. 12 is a fragmentary cross-section of a stent and balloon similar to that shown in FIGS. 10-11 taken along the longitudinal axis of the balloon, the stent in an expanded state and the balloon in a contracted state within a body vessel prior to withdrawal of the balloon.
FIG. 13 is a longitudinal side view of a stent disposed on a balloon and having a coating disposed over both the stent and the balloon representing not an embodiment of the present invention.
FIG. 14 is an exploded fragmentary cross-section taken at 14 in FIG. 13 showing a therapeutic agent(s) disposed between stent struts representing not an embodiment of the present invention.
FIG. 15 is a fragmentary cross-section of a stent and balloon taken along the longitudinal axis of the balloon illustrating an alternative embodiment of the coating according to the invention.
FIG. 16 is a fragmentary cross-section of a stent and balloon similar to that shown in FIG. 15, with the stent in an expanded state and in contact with a vessel wall.
FIG. 17 is a fragmentary cross-section of a stent and a balloon similar to that shown in FIGS. 15 and 16 illustrating another embodiment according to the invention.
FIG. 18 is a fragmentary cross-section of a stent and balloon similar to that shown in FIG. 17, with the stent in an expanded state and in contact with a vessel wall.
FIG. 19 is a partial longitudinal view of a coating employed in combination with a self-expanding stent and delivery system.
FIG. 20 is a partial longitudinal cross-sectional view of another embodiment of a coating employed in combination with a self-expanding stent and delivery system according to the invention.
FIG. 21 is a partial longitudinal cross-sectional view is a partial longitudinal cross-sectional view of another embodiment of a coating employed in combination with a self-expanding stent and delivery system representing not an embodiment of the present invention.

### DETAILED DESCRIPTIONS OF THE PREFERRED EMBODIMENTS

While this invention may be embodied in many different forms, there are described in detail herein specific preferred embodiments of the invention. This description is an exemplification of the principles of the invention and is not intended to limit the invention to the particular embodiments illustrated.

In one aspect, the present invention relates to novel coatings for medical devices. The novel coatings may find utility on any type of intraluminal medical device including, but not limited to, any type of catheter assembly or component thereof, stents, stent-grafts, grafts, vena cava filters, embolization devices, medical balloons, etc.

Examples of the various types of catheter assemblies include, but are not limited to, guide catheters, catheter for delivery of medical devices, diagnostic catheters, etc.

Catheter assemblies including those used for the delivery of other medical devices such as stents, are employed in a variety of body lumens including those found in the vascular system, biliary system, neurological system, reproductive system, urinary system, gastrointestinal system, etc.

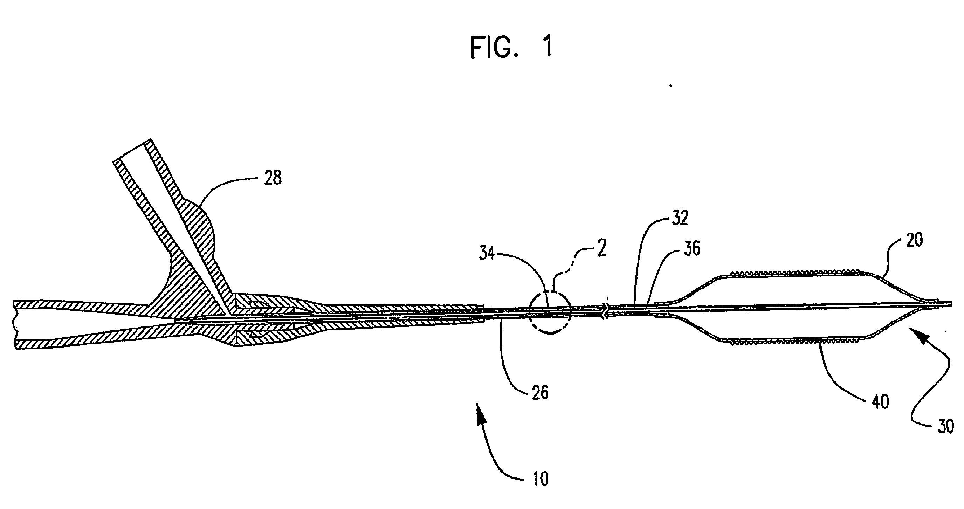

FIG. 1 is a longitudinal cross-sectional side view of a catheter assembly 10 according to the invention. Balloon 20 is mounted on the distal end 30 of catheter 10. A balloon expandable stent 40 is disposed on balloon 20.

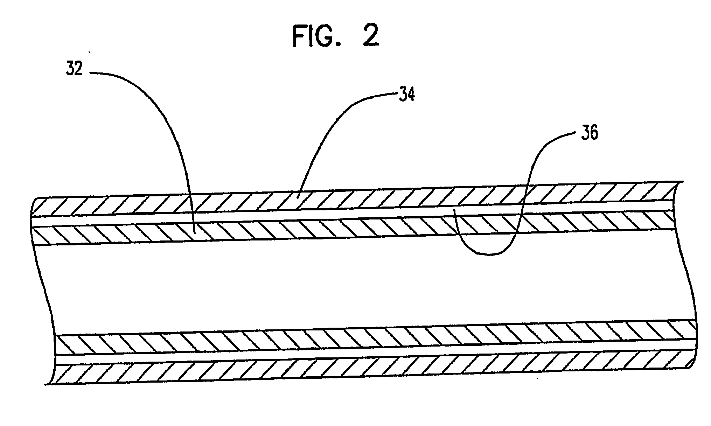

Catheter 10 is a representative simple over-the-wire (OTW) or single-operator-exchange (SOE) balloon catheter according to the invention. Such balloon catheters are discussed are well known. In this embodiment, catheter 10 has an elongate shaft assembly 26 and a conventional OTW-type manifold assembly 28 connected to proximal end of shaft assembly 26. The shaft assembly 26 includes an inner shaft 32 and an outer shaft 34. Outer shaft 34 is coaxially disposed about inner shaft 32 to define an annular inflation lumen 36 shown in enlarged fragmentary cross-section in FIG. 2 which is taken at section 2 in FIG. 1. Balloon 20 may be inflated by passing inflation fluid through manifold 28 resulting in deployment of stent 40. Negative pressure may then be applied to deflate and contract balloon 20. Procedures of this type are known in the art. Other catheter configurations are known which may also be employed herein. The invention is not limited by the type of catheter illustrated above.

The novel coatings according to the invention may be applied to balloon 20, stent 40 or a combination thereof. Furthermore, as described in various embodiments below, the novel coatings according to the invention may be applied to an inner member of a catheter delivery assembly employed in combination with self-expanding intraluminal medical devices.

The coatings herein are suitably degradable. In a typical embodiment, the coating shall be selected so as to degrade within an environment within a patient's body. This degradation may occur through any mechanism such as by at least partial dissolution as in an aqueous environment, or by a weakening of an ionic bond, hydrogen bond, van der Waals forces, or weakening of some other interaction. The invention is not limited by the type of mechanism which results in degradation or weakening of the coating.

This term degradation may also refer to decomposition wherein one substance breaks down into two simpler substances.

In an embodiment wherein a stent is disposed about the expandable member of a catheter assembly for deployment of the stent in a body vessel, the force of expansion and contraction of the expandable member can provide enough force to result in destruction of the coating integrity by separation of the layers in the case of an anionic/cationic LbL coating, for example. In this case, the coating can maintain the stent on the balloon for any suitable time up until deployment when the force provided by expansion and contraction of the expandable member results in a breaking of a weak ionic bond.

In another embodiment the coatings according to the invention are employed to help in securement of a self-expanding intraluminal medical device to an inner member of a catheter delivery assembly. The coating the coating degrades sufficiently in the body vessel that the stent is readily released from the inner member upon expansion of the self-expanding stent.

The coatings according to the invention may be designed such that the coating degrades over seconds, minutes, or days.

In one embodiment wherein a degradable coating is employed which dissolves in an aqueous environment, the coating may rapidly weaken, as within seconds or minutes. This weakening may also be enhanced by the increase in surface area upon expansion of the expandable balloon member and the stent.

Any suitable degradable material can be employed in the coatings according to the invention. Examples of suitable materials include, but are not limited to, those that are water soluble, dispersible, dissolvable, sensitive, etc. As used herein, the term "water soluble" shall include those materials which have partial solubility in water. Hereinafter, the term "hydrophilic" shall be used to refer to any materials having these various degrees of water sensitivity.

Suitable polymers of this type which are useful herein are typically non-crosslinked structures having hydrophilic groups thereon such as -OH, -COOH, -CONH, -COO-, etc. Of course, the simple presence of such groups does not insure that the polymer is hydrophilic. It will also depend on the polymer structure, the number of such groups, etc.

Examples of suitable hydrophilic polymers include, but are not limited to, polyalkylene glycols such as polyethylene glycol (PEG) and modified polyethylene glycols, polyethylene oxide and hydrophilic block copolymers of polyethylene oxide and polypropylene oxide, carbohydrates, sugar alcohols such as mannitol, polyols, monosaccharides, oligosaccharides, polysaccharides and modified polysaccharides such as Heparin (mucopolysaccharide), hydrophilic polyurethanes such as polyether aliphatic polyurethanes, hydrophilic polyamides, hydroxyethyl methacrylate (HEMA), salts of polyacrylic acid such as the alkali metal salts (Na, K are the most common) or alkaline earth metal salts of polyacrylic acid, polyvinyl alcohol, polyvinyl acetate, polyvinylpyrrolidone (a hydrophilic poly(N-vinyl lactam), cellulose and hydrophilic modifications thereof such as carboxymethyl cellulose, methyl cellulose, hydroxyethyl cellulose and hydroxypropyl cellulose, methyl vinyl ether-maleic anhydride copolymers, proteins, peptides, DNA, etc.

Hydrophilic polymers are discussed in commonly assigned U.S. Patent No. 5509899 to Fan et al.

These hydrophilic polymers may be applied to the medical device as a single layer, or they may be applied in multiple layers.

Preferable hydrophilic polymers for use herein are those which rapidly dissolve in an aqueous environment such as polyethylene glycol, mono-, oligo- and polysaccharides and modified polysaccharides, carbohydrates, sugar alcohols such as mannitol, and polyols, for example. Desirably, the coating material is biocompatible.

Ionic materials and mixtures thereof may also be employed in the degradable coatings according to the invention.

In one embodiment, the coating according to the invention is employed for the purposes of stent securement. In the case of a coating for stent securement, the coating shall degrade or weaken enough that the stent is readily released from the balloon upon contraction of the balloon.

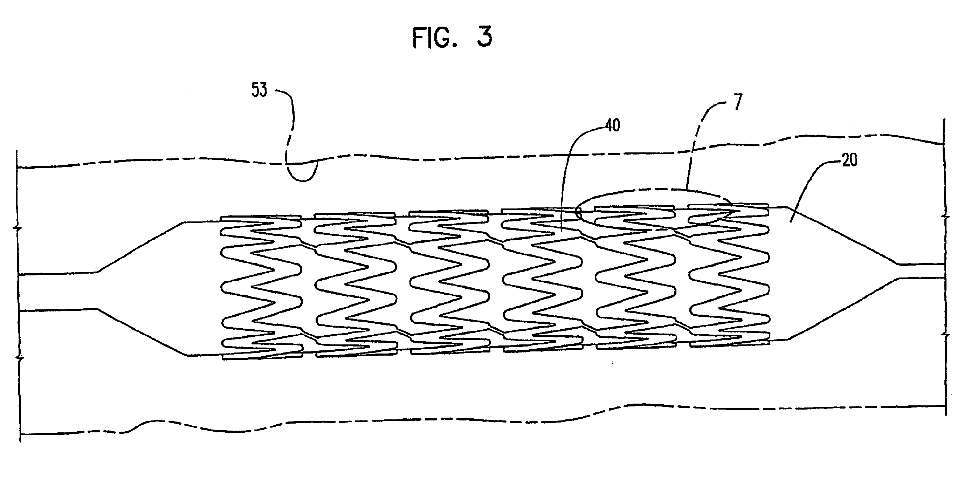

FIGS. 3-6 illustrate not an embodiment of the invention wherein a single, layer of a degradable polymeric coating is applied over a stent and balloon. Suitably, the layer is ultrathin. For example, in the case of a LbL coating, each layer may have a thickness in the nanometer range. For a degradable coating for which the coating actually separates from itself once it is weakened, the thickness may be in the micrometer range. Thus, coating thicknesses may range from about 1 nanometer up to about 20 micrometers, suitably about 10 nanometers up to about 10 micrometers.

FIG. 3 is a longitudinal side view of an expandable balloon member 20 having a stent 40 disposed thereon. Stent 40 is shown in a crimped state. The stent shown in FIG. 3 is for illustrative purposes only. The stent may be of any configuration known in the art and may vary depending on the type of medical procedure for which it is being employed.

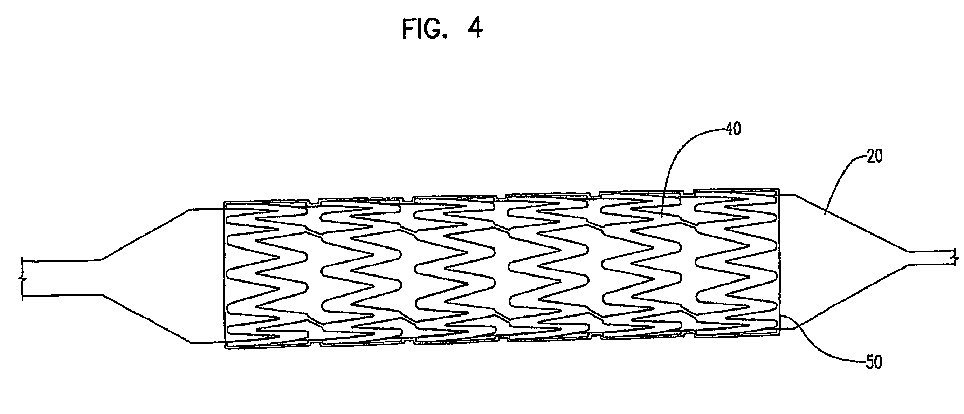

FIG. 4 is a longitudinal side view of an expandable balloon member 20 having stent 40 disposed thereon. A degradable coating 50 is shown disposed over both the stent 40 and the expandable balloon member 20. Suitable examples of degradable coatings were presented for illustrative purposes, above.

The coating may be disposed over only a portion of the stent 40 and only a portion of the expandable balloon 20 as well.

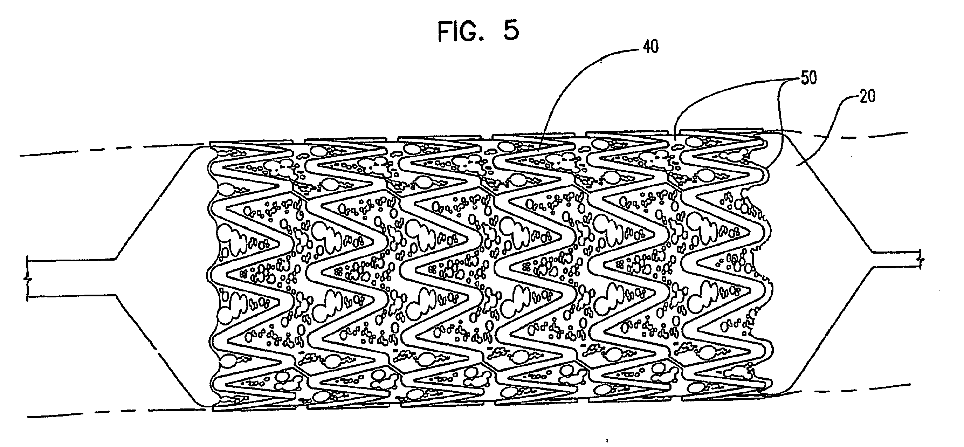

FIG. 5 is a longitudinal side view of balloon 20 and stent 40 disposed on the balloon. Balloon 20 has been inflated and stent 40 expanded. This is typically done at the site of deployment of the stent once the stent has been positioned at the desired location in the body lumen. Suitably, degradable coating 50 begins to weaken through a mechanism as described herein, such as by dissolution. This is enhanced by the fact that upon expansion of the balloon and stent, the surface area of the coating is greatly enlarged.

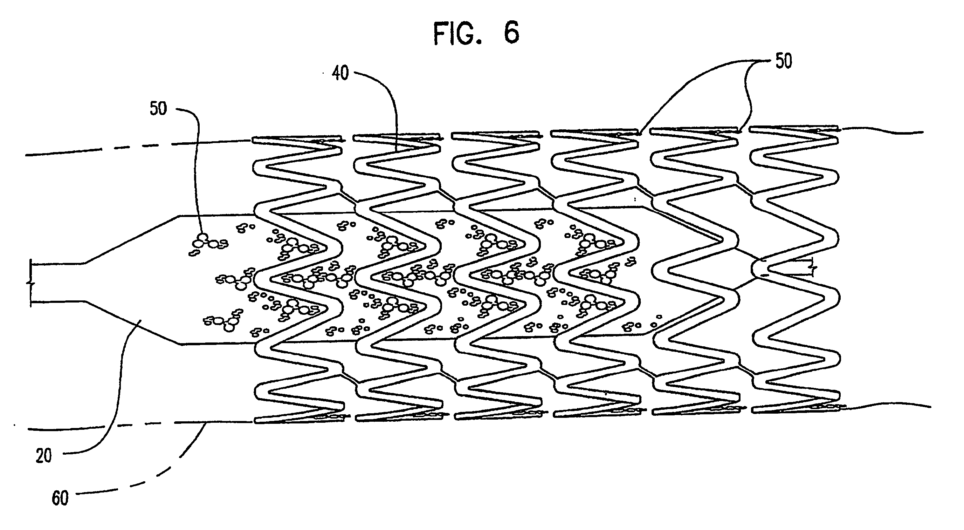

FIG. 6 is a longitudinal side view of balloon 20 shown in a partially contracted or deflated state and stent 40 which remains deployed in the vessel in an expanded state. Balloon 20 may be contracted using any method known in the art such as through the application of negative pressure to remove fluid from the annular lumen. Coating 50, now in an at least partially degraded state, is shown on both balloon 20 and stent 40. Balloon 20 may be withdrawn from a body lumen once contracted.

The coating according to the invention is fabricated in multi-layer films assembled through the sequential absorption of oppositely charged species during a stepwise absorption from solution. These coatings are referred as layer-by-layer (LbL) coatings. See, for example, Polyelectrolyte multilayer capsule permeability control, Antipov, Alexei A. et al., Colloids and Surfaces A: Physiochemical and Engineering Aspects 198-200, Elsevier Science B.V. (2002), pp. 535-541 and Incorporation of macromolecules into polyelectrolyte micro- and nanocapsules via surface controlled precipitation on colloidal particles, Radtchenko, Igor L. et al., Colloids and Surfaces A: Physiochemical and Engineering Aspects 202, Elsevier Science B.V. (2002), pp. 127-133.

Alternatively, polyelectrolyte complexes in the form of a soluble ink can be applied. An example is found in Phase Behavior and Rheological Properties of Polyelectrolyte Inks for Direct-Write Assembly, Gratson, Gregory M. and Lewis, Jennifer A., Langmuir 21 (2005), pp. 457-464.

Suitable materials for use in LbL coatings include, but are not limited to, polyelectrolytes, proteins, DNA, inorganic particles, lipids, and so forth.

Ionic polymers may be suitably employed in the multi-layer coatings according to the invention. The ionic polymers may be anionic or cationic in nature and may include but are not limited to carboxylic, sulfate, and amine functionalized polymers such as polyacrylic acid, polymethacrylic acid, polyethylene amine, polysaccharides such as alginic acid, pectinic acid, carboxy methyl cellulose, hyaluronic acid, heparin (mucopolysaccharide), chitosan, carboxymethyl chitosan, carboxymethyl starch, carboxymethyl dextran, heparin sulfate, chondroitin sulfate, cationic guar, cationic starch, and their salts. Preferred ionic polymers are alginic acid, pectinic acid, carboxymethyl cellulose, hyaluronic acid, chitosan, and their salts. Most preferred ionic polymers are alginic acid, pectinic acid, and hyaluronic acid and their salts. As previously noted, the ionic polymers employed in the present invention are categorized as anionic polymers and cationic polymers. Among the anionic polymers that may be employed are polyacrylic acid, polymethacrylic acid, alginic acid, pectinic acid, carboxy methyl cellulose, hyaluronic acid, heparin, carboxymethyl starch, carboxymethyl dextran, heparin sulfate, and chondroitin sulfate. Among the cationic polymers that may be employed are chitosan, cationic guar, cationic starch and polyethylene amine.

The above list is intended for illustrative purposes only and not to limit the scope of the present invention. Such polymers are known to those of skill in the art.

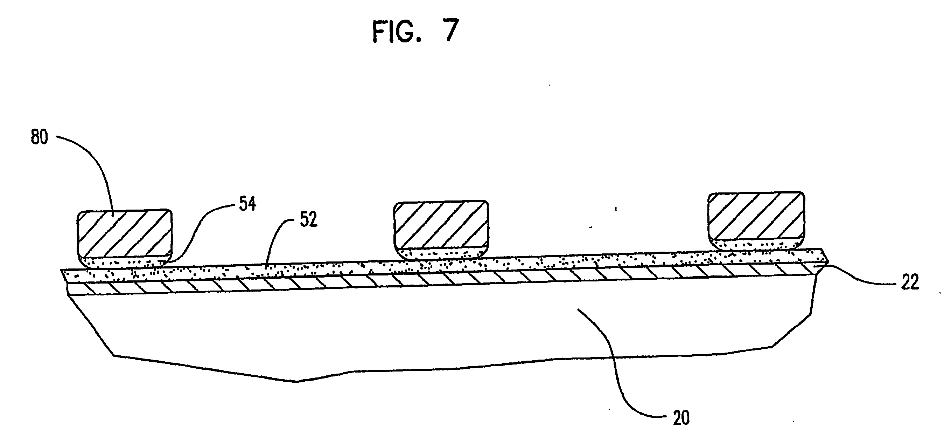

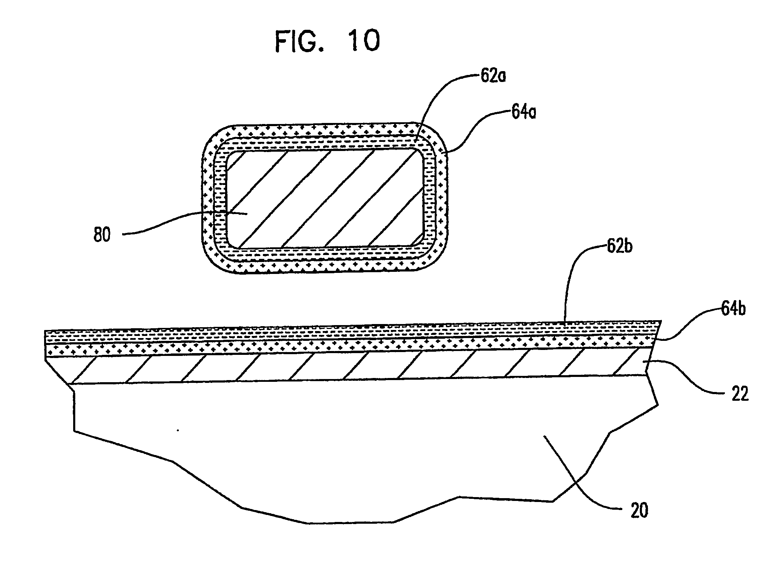

FIGS. 7-9 illustrate an embodiment of the invention wherein a layer-by-layer (LbL) coating having at least one layer having a material with a negative charge (anionic) and at least one second layer having a material with a positive charge (cationic) is disposed on the balloon and the stent. In the embodiment shown in FIGS. 7-9, one layer is disposed on the balloon and one layer disposed on the stent. However, this is only an illustration of the invention. Both layers may be disposed on the balloon or both layers disposed on the inner surface of the stent, or both layers may be disposed over both the stent and the balloon, or one layer on the balloon and one layer disposed over the stent, etc. Furthermore, multiple layers may be disposed on each of the stent and the balloon as well. An example of such an embodiment is illustrated in FIGS. 10-12 below.

FIG. 7 is a fragmentary section taken along the longitudinal axis of balloon 20 at section 7 in FIG. 3. Wall 22 of medical balloon 20 is shown having a coating 52 disposed thereon. Coating layer 52 may include either a cationic material or an anionic material. Struts 80 of a stent are shown having a coating 54 disposed thereon. Coating layer 54 may include either a cationic material or an anionic material providing it has the opposite charge of coating layer 52.

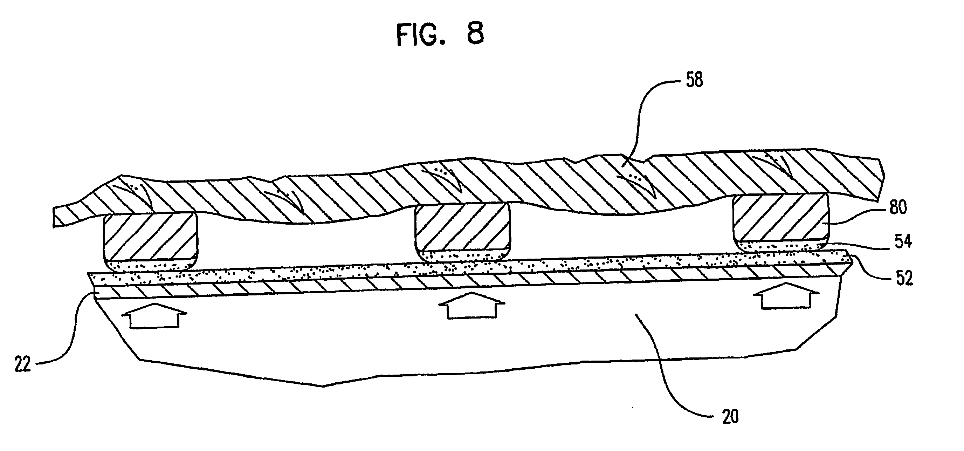

FIG. 8 is a fragmentary section taken along the longitudinal axis of balloon 20 having a stent disposed thereon. This view shows the balloon/stent combination after insertion into a body lumen and shown with stent struts 80 in contact with vessel wall 58. Balloon 20 has been inflated and the stent expanded.

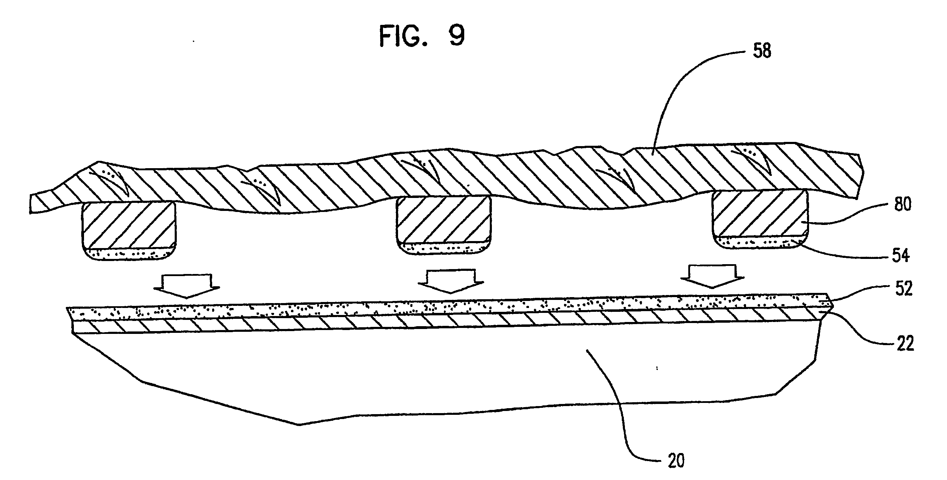

Balloon 20 is then contracted, typically through application of a negative pressure. The weak ionic bond formed between coating layer 52 and coating layer 54, is broken at this point as shown in FIG. 9, releasing the stent from the balloon 20. Furthermore, the increase in surface area also results in weakening of the electrochemical forces between layers 52 and 54.

Other types of materials which form weak hydrogen bonding, or are attracted through van der Waals forces may also be employed herein. Any type of materials which form chemical bonds which can be broken either through mechanical forces or through physico-chemical means as described above, may be employed herein.

A specific example of a combination of anionic/cationic materials which may be employed herein is chitosan and heparin. An ionic bond between the chitosan and heparin molecules is sufficient to hold the stent in place on the balloon during delivery of the stent through a body lumen to the site of deployment. Upon expansion and/or contraction of the expandable medical balloon, breaks may occur in the coating, allowing wide spread aqueous penetration. The ionic bond formed between the heparin molecules and the chitosan molecules breaks, thus releasing the stent from the expandable medical balloon.

The following structure is representative of a sulfated heparin molecule, although the exact structure is uncertain:

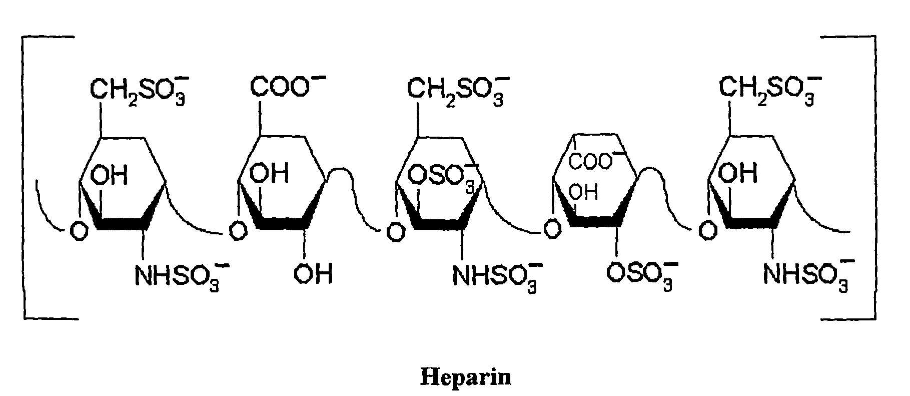

Chitosan is a polysaccharide consisting of (1-4)-linked 2-amino-2-deoxy--D-glucopyranose. Chitosan is cationic in nature in acidic solutions, as compared to many other polysaccharides which are negatively charged.

Chitosan has the following general structure:

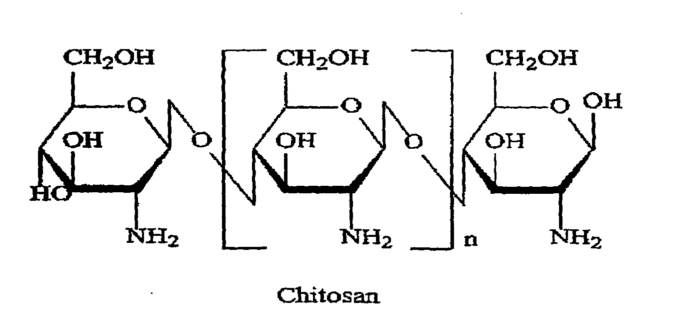

Chitosan can also be sulfated. Chitosan polysulfate dissolves very well in aqueous environments.

Chitosan and heparin are biocompatible materials.

In an alternative embodiment shown in fragmentary cross sections in FIGS. 10-12 which are taken along the longitudinal axis of the balloon, multiple layers having cationic and anionic material may be employed. In this embodiment, stent strut 80, as shown in FIG. 10, has a layer 62a including an anionic material, and disposed thereon is an outer layer 64a including a cationic material. The coating layers may be disposed on the stent using any method known in the art such as by dipping, spraying, painting, etc. Disposed on balloon wall 22 is a layer 64b including a cationic material followed by an outer layer 62b including an anionic material. The coating layers may also be disposed on the balloon using any method known in the art. In other embodiments, ionic materials or mixtures thereof may be employed as a single coating layer as discussed above.

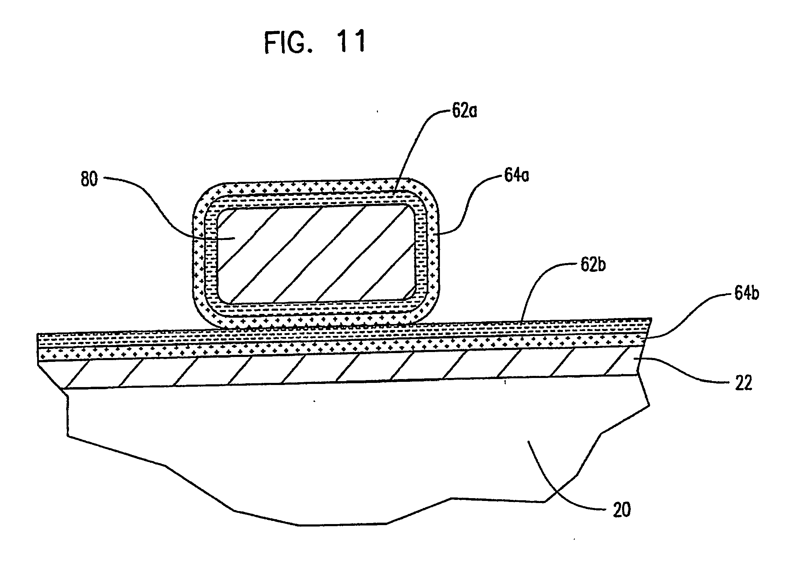

The stent may be crimped onto balloon 20 as known in the art forming a weak ionic bond between outer layer 64a (cationic) on stent strut 80 and outer layer 62b (anionic) on balloon wall 22 shown as a fragmentary section taken along the longitudinal axis of balloon 20 in FIG. 11.

The assembly may then be inserted into a body lumen and maneuvered to the site of deployment in a body vessel, the balloon inflated thereby expanding and the stent (not shown) as known in the art. The balloon is then contracted and the stent released.

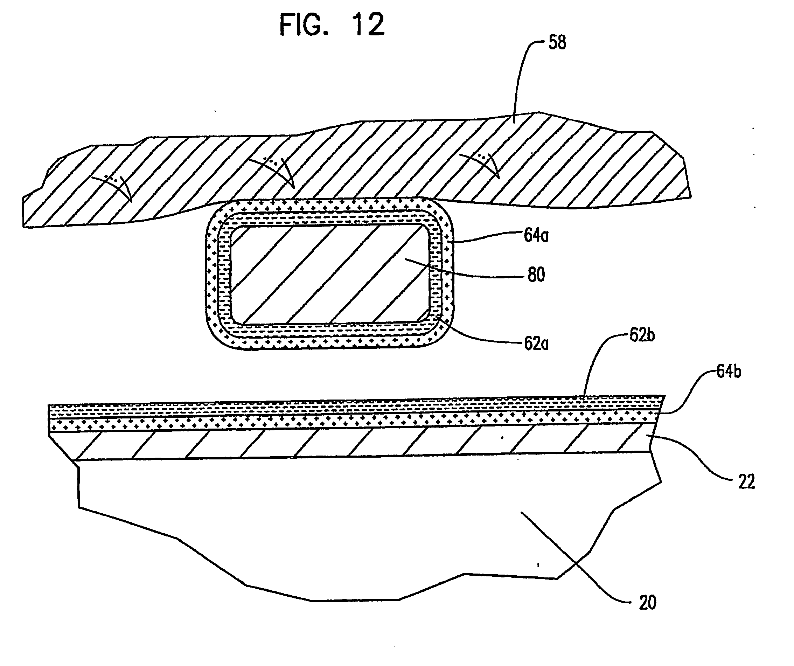

FIG. 12 is a fragmentary sectional view taken along the longitudinal axis of balloon 20. Stent strut 80 is shown in contact with body vessel 58 after inflation of balloon 20 and expansion of the stent. The weak ionic bond between coating layer 64a (cationic) and coating layer 62b (anionic) has been easily broken in the course of deployment of the stent and contraction of the balloon. This weakening of the electrostatic forces is also enhanced by the increase in the surface area of the balloon and stent during expansion.

The above embodiment described in FIGS. 10-12 is only one illustration of a multi-layer construction according to the invention. The order of the cationic/anionic coating layers may be varied, providing that at least one anionic layer is adjacent at least one cationic layer such that the ionic bond may be broken upon stent expansion and/or balloon contraction.

Furthermore, other multilayer constructions having more than two layers are within the scope of the invention. For example, ten layers may be applied with the weak bond formed between layers five and six. Thus, multiple layers may be employed providing there are adjacent anionic/cationic layers for which the ionic bond may be broken and the layers split.

Therapeutic agent(s) may be optionally employed herein. "Therapeutic agents," "drugs," "pharmaceutically active agents," "pharmaceutically active materials," and other related terms are employed in the art interchangeably. Hereinafter, the term therapeutic agent will be employed herein. Therapeutic agents include genetic materials, non-genetic materials, and cells.

The therapeutic agent or mixtures thereof, may be included in a polymeric coating layer, or in some instances, the therapeutic agent itself may be applied as a layer. For example, heparin, itself a therapeutic agent, may be employed as a coating layer as described above.

The therapeutic agent(s) may be exposed to the surrounding environment either upon splitting of a LbL coating or through degradation/destruction of the coating.

Examples of non-genetic therapeutic agents include, but are not limited to, anti-thrombogenic agents, anti-proliferative agents, anti-inflammatory agents, analgesics, antineoplastic/antiproliferative/anti-miotic agents, anesthetic agents, anti-coagulants, vascular cell growth promoters, vascular cell growth inhibitors, cholesterol-lowering agents; vasodilating agents; and agents which interfere with endogenous vascoactive mechanisms.

Genetic agents include anti-sense DNA and RNA and coding DNA, for example.

Cells may be of human origin, animal origin, or may be genetically engineered.

Examples of anti-thrombogenic agents include, but are not limited to, heparin, heparin derivatives, urokinase, and PPack (dextrophenylalanine proline arginine chloromethylketone).

Examples of anti-proliferative agents include, but are not limited to, enoxaprin, angiopeptin, or monoclonal antibodies capable of blocking smooth muscle cell proliferation, hirudin, acetylsalicylic acid, to mention only a few.

Examples of anti-inflammatory agents include steroidal and non-steroidal anti-inflammatory agents. Specific examples of steroidal anti-inflammatory agents include, but are not limited to, budesonide, dexamethasone, desonide, desoximetasone, corticosterone, cortisone, hydrocortisone, prednisolone, to mention only a few.

Specific examples of non-steroidal anti-inflammatory agents include, but are not limited to, acetylsalicylic acid (i.e. aspirin), ibuprofen, ibuproxam, indoprofen, ketoprofen, loxoprofen, miroprofen, naproxen, oxaprozin, piketoprofen, pirprofen, pranoprofen, protizinic acid, sulfasalazine, mesalamine, suprofen, tiaprofenic acid, to mention only a few.

Examples of analgesics include both narcotic and non-narcotic analgesics. Examples of narcotic analgesics include, but are not limited to, codeine, fentanyl, hydrocodone, morphine, promedol, to mention only a few.

Examples of non-narcotic analgesics include, but are not limited to, acetaminophen, acetanilide, acetylsalicylic acid, fenoprofen, loxoprofen, phenacetin, to mention only a few.

Examples of antineoplastic/antiproliferative/anti-miotic agents include, but are not limited to, paclitaxel, 5-fluorouracil, cisplatin, vinblastine, vincristine, epothilones, endostatin, angiostatin and thymidine kinase inhibitors.

Examples of anesthetic agents include, but are not limited to, lidocaine, bupivacaine, and ropivacaine, to mention only a few.

Examples of anti-coagulants include, but are not limited to, D-Phe-Pro-Arg chloromethyl keton, an RGD peptide-containing compound, heparin, antithrombin compounds, platelet receptor antagonists, anti-platelet receptor antibodies, aspirin, prostaglandin inhibitors, platelet inhibitors and tick antiplatelet peptides.

Derivatives of many of the above mentioned compounds also exist which are employed as therapeutic agents.

Of course mixtures of any of the above may also be employed.

The above lists are intended for illustrative purposes only, and not as a limitation on the scope of the present invention.

Therapeutic agents are discussed in commonly assigned U.S. Patent Application 20040215169, the entire content of which is incorporated by reference herein.

In the case where an LbL coating is employed, one or more layers may be a therapeutic agent such as, for example, where heparin is employed as a layer on the balloon or stent.

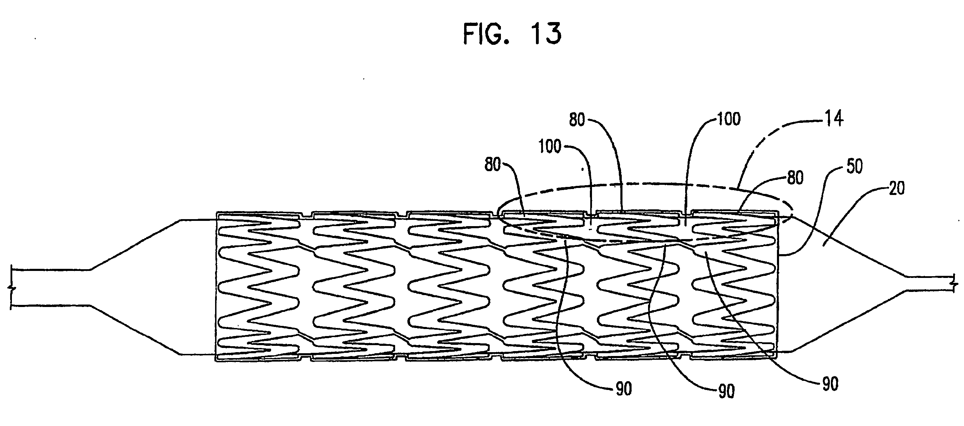

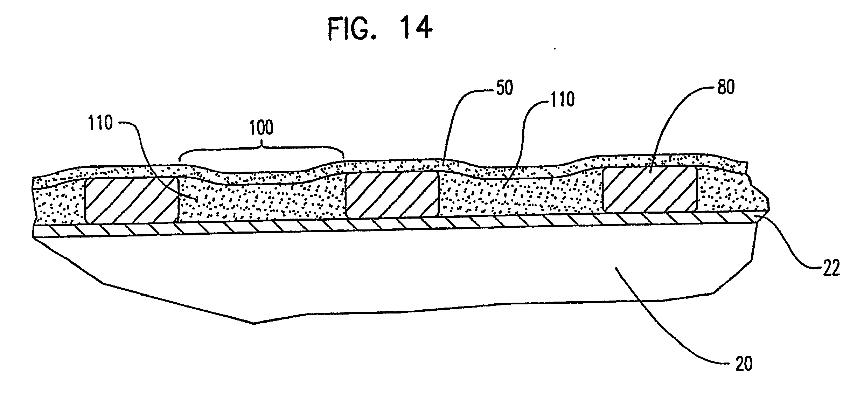

FIGS. 13 and 14 are representative of an example wherein therapeutic agent(s) are employed. FIG. 13 is a longitudinal side view of an expandable balloon member 20 having a stent 40 disposed thereon. Coating 50 is disposed over both stent 40 and balloon 20. Stent 40 has a strut pattern having a plurality of struts 80 and end portions 90 which define a plurality of openings 100. At least a portion of one of more of openings 100, may have a therapeutic agent(s) disposed therein. This is shown as an enlarged fragmentary view in FIG. 14 which is taken along the longitudinal axis of balloon 20 at section 14 in FIG. 13. A degradable coating 50 is disposed over stent and balloon enclosing therapeutic agent(s) 110.

The stent configuration shown in FIGS. 13-14 is for illustrative purposes only. Any suitable stent configuration may be employed herein.

In this embodiment, upon exposure to a polar, for example, an aqueous environment, the coating degrades, allowing the therapeutic agent to be released. The rate of release may be controlled by the type of degradable coating selected. For example, highly hydrophilic coatings, such as those having polyethylene glycol, polyvinyl alcohol, or some such polymer, may dissolve quickly, allowing therapeutic agent to escape.

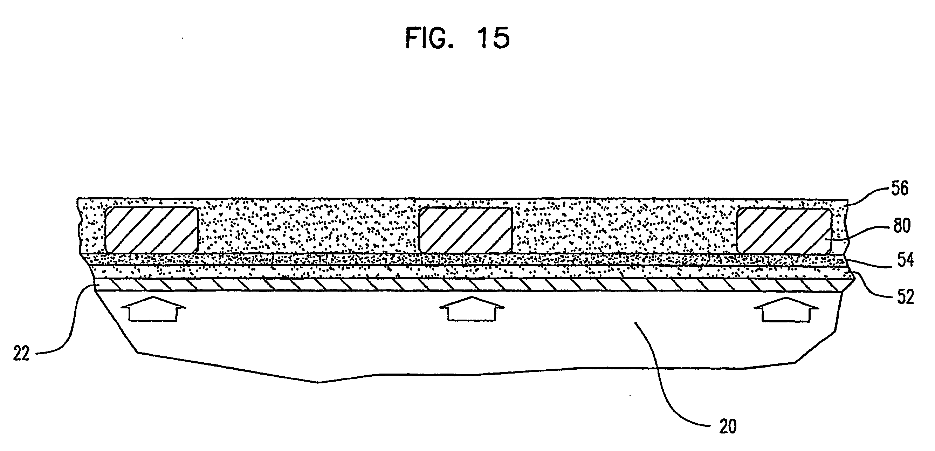

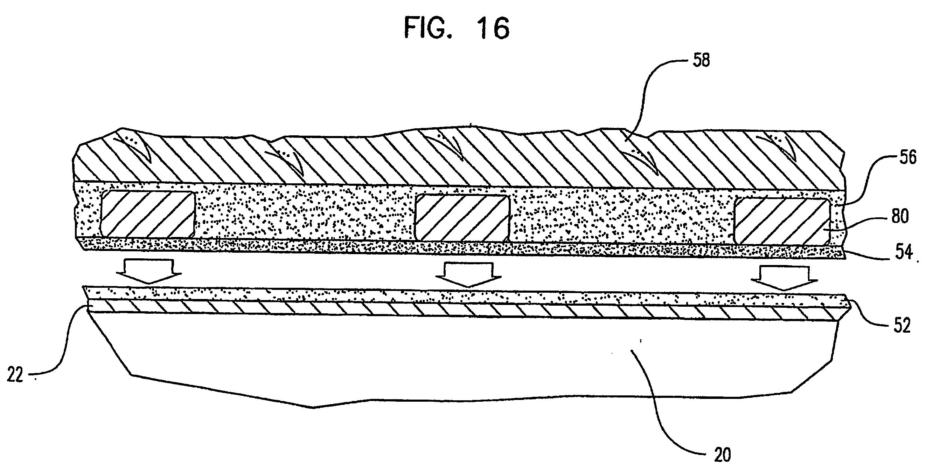

An alternative embodiment of the degradable coatings employed in combination with therapeutic agent(s) is shown as fragmentary cross-sections in FIGS. 15 and 16. Balloon 20 has disposed on the outer surface of wall 22, a first coating layer 52 having a material which is either cationic or anionic and a second coating layer 54 having a material with the opposite charge as that of the first coating layer 52. The layers may be interchanged, providing that each layer has a material of the opposite charge such that an ionic bond can be formed between the layers. A third coating layer 56 having a material of the opposite charge as that of second coating layer 54 may be applied after the stent has been crimped on the balloon.

Thus, in one embodiment, first coating layer 52 includes an anionic material, second coating layer 54 includes a cationic material and third coating layer 56 includes an anionic material.

In another embodiment, first coating layer 52 includes a cationic material, second coating layer 54 includes an anionic material and third coating layer 56 includes a cationic material..

Third coating layer 56 may also include at least one therapeutic agent or mixture of therapeutic agents. Suitably, the ionic bond formed between first coating layer 52 and second coating layer 54 is weaker than the ionic bond formed between second coating layer 54 and third coating layer 56 such that when the stent is deployed within a body vessel, the LbL coating layers split between layers 52 and 54, leaving coating layer 56 with the therapeutic agent or mixtures thereof, trapped between coating layer 54 and the vessel wall as shown as a fragmentary cross-section in FIG. 16.

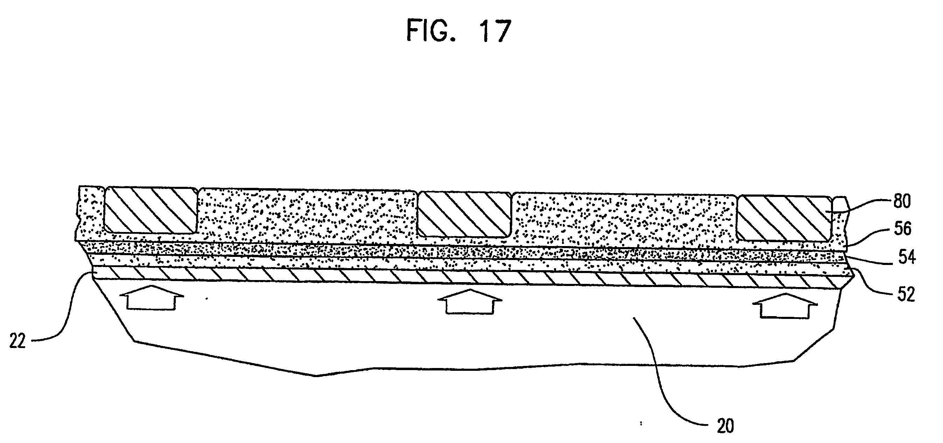

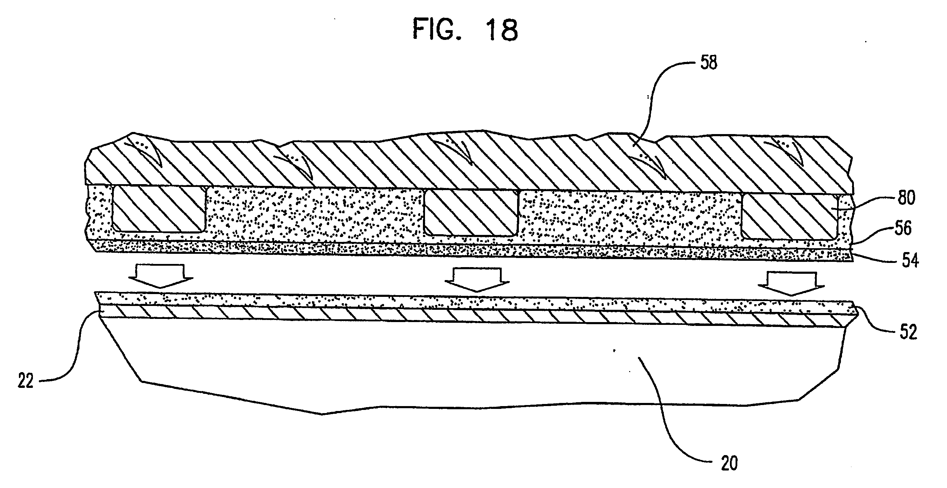

Alternatively, the third coating layer 56 may be applied to the balloon 20 prior to crimping the stent onto the balloon 20 as shown as a fragmentary cross-section in FIG. 17. The weak bond is formed between layers 52 and 54 such that when the stent is expanded, layer 56 is trapped between layer 54 and the vessel wall 58 as shown as a fragmentary cross-section in FIG. 18.

The degradable coatings according to the invention may be employed in combination with other types of coatings known in the art including, for example, drug eluting coatings. In one such embodiment, a degradable coating according to the invention may be employed as an intermediate coating between a balloon and a stent having a drug eluting coating in order to reduce adhesion which may occur between the drug eluting coating and the balloon on which the stent is crimped upon expansion and deployment of the stent.

Examples of polymer materials employed in a drug eluting layer include, but are not limited to, block copolymers such styrenic block copolymers. Examples of styrenic block copolymers include, but are not limited to, styrene-isoprene-styrene (SIS), styrene-butadiene-styrene (SBS), styrene-ethylene/butylene-styrene (SEBS), styrene-ethylene/propylene-styrene (SEPS), styrene-isobutylene-styrene (SIBS), etc.

Therapeutic agent(s), as discussed above, may be employed in combination with such polymers to form a drug eluting layer.

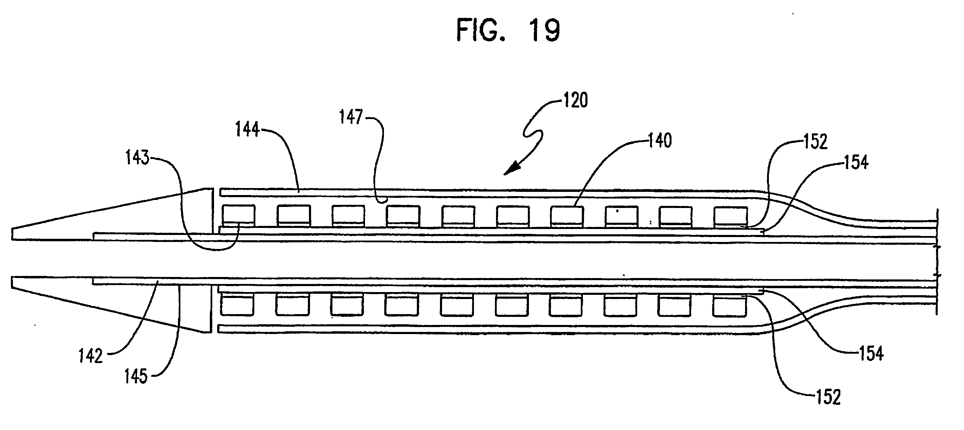

In another embodiment, a degradable coating according to the invention is employed in a self-expanding stent delivery system 120 shown as a partial longitudinal cross-section of the distal end of the delivery system 120 in FIG. 19. Self-expanding stent 140 is shown disposed on inner member 142 with a reduced diameter configuration, and is secured with stent securement sheath 144.

In one embodiment, a first coating layer 152 is disposed on the inner surface 143 of stent 140 and a second coating layer 154 is shown disposed on the outer surface 145 of inner member 142. First coating layer 152 includes a material carrying either a positive charge or a material carrying a negative charge and second coating layer 154 includes a material carrying the opposite charge as that of coating layer 152. An ionic bond can thus be formed between coating layer 152 and coating layer 154 in order to facilitate securement of the stent 140 to the inner member 142 during delivery of the stent 140 to the site of deployment with a patient's body vessel.

In a typical self-expanding stent delivery system, stent 140 can exert force upward onto the inner surface 147 of stent securement sheath 144 and can imprint on the inner surface resulting in the need for a higher axial force when the sheath 144 is pulled back to release the stent 140 at the site of deployment.

In the embodiment described above the ionic attraction between coating layer 152 and coating layer 154 helps to secure stent 140 during delivery thereby helping to reduce the radial force of the stent against the sheath. The coatings according to the invention will also help to reduce the axial force required when the sheath 140 is pulled back to release the stent 140. Upon exposure to the environment within the body vessel, and with mechanical force exerted by the stent during expansion after pulling sheath 140 back to release stent 140, the ionic bond between coating layer 152 and coating layer 154 breaks, releasing the stent 140 from the inner member 142.

While the embodiment described above is specific to ionic systems, other types of degradable coatings may be employed herein. Coatings may be selected so that degradation occurs within the body. For example, degradation may occur by at least partial dissolution in an aqueous environment, by weakening of hydrogen bonding, by weakening of van der Waals forces, or by a weakening of some other interaction. The invention is not limited by the type of mechanism which results in degradation or weakening of the coating.

For example, in another embodiment, the coating is water sensitive, thereby degrading sufficiently upon exposure to an aqueous environment that stent 140 may release from the inner member 142.

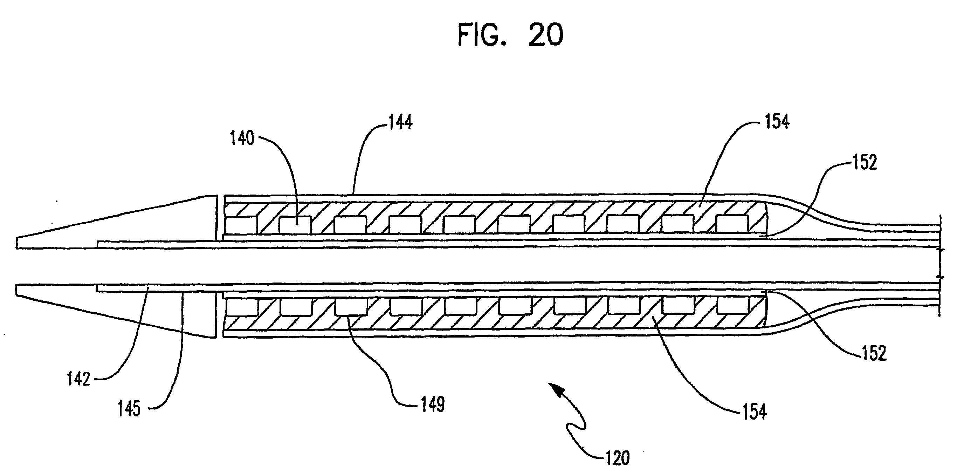

FIG. 20 is a partial longitudinal cross-sectional view of the distal end of a self-expanding stent delivery system 120. Self-expanding stent delivery systems are known in the art. Self-expanding stent 140 is shown disposed on inner member 142 in a reduced diameter configuration and with stent securement sheath 144 securing the stent 140 to the inner member 142. A first coating layer 152 is disposed on the outer surface 145 of inner member 142. First coating layer 152 may include either a material carrying a positive charge or a material carrying a negative charge. A second coating layer 154 is disposed on the outer surface 149 of stent 140. Second coating layer 154 includes a material which carries the opposite charge to that of the material included in the first coating layer 152 such that an ionic bond is formed between first coating layer 152 and second coating layer 154. This LbL coating helps decrease the axial force required to pull the sheath 144 back from stent 140 during deployment as described above. When the sheath 144 is pulled back, the stent 140 is allowed to expand. The combination of exposure to an aqueous environment and the mechanical force provided during stent expansion, results in a separation between the first coating layer 152 and the second coating layer 154.

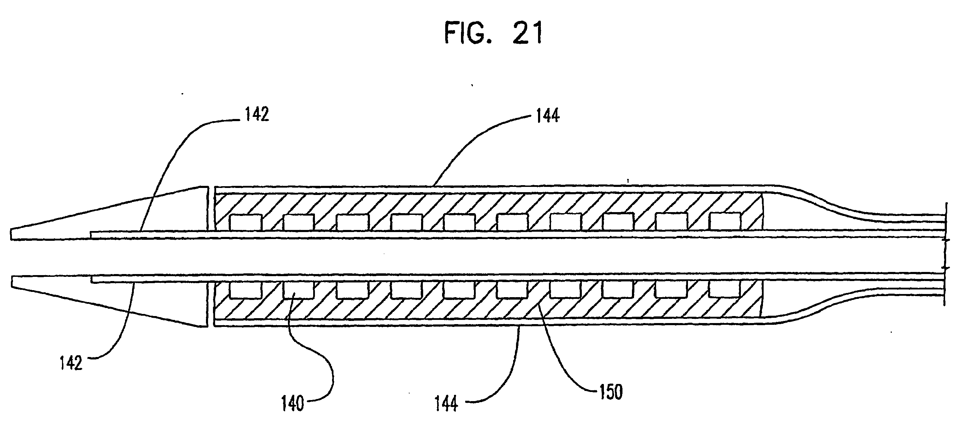

FIG. 21 is a partial longitudinal cross-sectional view of the distal end of a self-expanding stent delivery system 120, illustrating an alternative example of a degradable coating 150 employed in such a delivery system 120. A sheath 144 is disposed over the stent to secure stent 140 to inner member 142. In this example, a single coating layer 150 is disposed over both stent 140 and inner member 142. Coating 150 is a degradable coating. Coating 150 helps secure stent 140 in a reduced diameter configuration to inner member 142. Again, as described above, coating 150 helps reduce the axial force required to pull sheath 144 back from stent 140 during deployment in a patient's body vessel. In this embodiment, upon exposure to an aqueous environment such as within a patient's body vessel, coating 150 begins to dissolve therefore weakening. The compromised integrity of the coating results in breakage upon expansion of the stent 140.

Such coatings have been described in detail above.

Some examples of preferable hydrophilic polymers for use in such an embodiment include those which rapidly dissolve in a polar or an aqueous environment such as polyethylene glycol, mono-, oligo- and polysaccharides and modified polysaccharides, carbohydrates, sugar alcohols such as mannitol, and polyols, for example. Desirably, the coating material is biocompatible.

The above disclosure is intended to be illustrative and not exhaustive. This description will suggest many variations and alternatives to one of ordinary skill in this art. All these alternatives and variations are intended to be included within the scope of the attached claims.

## Claims

1. A catheter assembly (10) comprising:
an expandable balloon member (20) having an inner surface and an outer surface disposed on the expandable balloon member;
an expandable intraluminal medical device (40) having an inner surface and an outer surface and being disposed on the expandable balloon member; and a degradable coating (50) in contact with at least a portion of the inner surface or outer surface of the expandable intraluminal medical device (40) and in contact with at least a portion of the outer surface of the expandable balloon member,
the degradable coating is a layer-by-layer coating (52, 54) selected so as to release the expandable intraluminal medical device from the expandable balloon member upon expansion or contraction of the expandable balloon member from an inflated state upon exposure to an environment within the body, the layer-by-layer coating comprising at least one first layer (52) and at least one second layer (54), said at least one first layer comprising a material carrying a negative charge or a material carrying a positive charge and said at least one second layer comprising a material carrying the opposite charge to that of the first layer.

2. The catheter assembly (10) of claim 1, wherein said first layer (52) or said second layer (54) is disposed on at least a portion of said outer surface of said expandable balloon member.

3. The catheter assembly (10) of claim 2, wherein said first layer (52) is disposed on at least a portion of said outer surface of said expandable balloon member (20) and said second layer (54) is disposed on at least a portion of said inner surface of said expandable medical device.

4. The catheter assembly (10) of claim 2, wherein said first layer (52) is disposed on at least a portion of said outer surface of said expandable balloon member (20), said second layer (54) is disposed on at least a portion of said outer surface of said expandable medical device (40).

5. The catheter assembly (10) of claim 1, wherein said at least one first layer (52) and at least one second layer (54) each comprise a member selected from the group consisting of polyelectrolytes, polyelectrolyte complexes, inorganic particles, inorganic polymers, inorganic lipids, ionic polymers, proteins, DNA and mixtures thereof.

6. The catheter assembly (10) of claim 1, wherein said at least one first layer (52) and at least one second layer (54) comprises an ionic polymer selected from the group consisting of carboxylic functionalized polymers, sulfate functionalized polymers, amine functionalized polymers, carbohydrates, monosaccharides, oligosaccharides, polysaccharides, polyols, sugar alcohols and mixtures thereof.

7. The catheter assembly (10) of claim 6, wherein said at least one first layer (52) and at least one second layer (54) comprise an ionic polymer selected from the group consisting of polyacrylic acid, polymethacrylic acid, polyethylene amine, polysaccharides, alginic acid, pectinic acid, carboxy methyl cellulose, hyaluronic acid, heparin, chitosan, carboxymethyl chitosan, carboxymethyl starch, carboxymethyl dextran, heparin sulfate, chondroitin sulfate, cationic guar, cationic starch, alginic acid, pectinic acid, hyaluronic acid, any salts thereof and mixtures thereof.

8. The catheter assembly (10) of claim 1, wherein said first layer (52) comprises heparin and said second layer (54) comprises chitosan.

9. The catheter assembly (10) of claim 1, wherein the degradable coating (52, 54) is selected so as to release the medical device (40) from the expandable balloon member (20) in an aqueous-based environment.

10. The catheter assembly (10) of claim 9, wherein the degradable coating (52, 54) comprises a material which is selected so as to dissolve in an aqueous-based environment.

11. The catheter assembly (10) of claim 1, wherein the degradable coating (52, 54) comprises at least one member selected from the group consisting of polyethylene glycol, modified polyethylene glycols, polyethylene oxide, block copolymers of polyethylene oxide and polypropylene oxide, polysaccharides, modified polysaccharides, hydrophilic polyurethanes, hydrophilic polyamides, hydroxyethyl methacrylate (HEMA), polyacrylic acid, polyvinyl alcohol, polyvinyl acetate, polyvinylpyrrolidone, cellulose, carboxymethyl cellulose, methyl cellulose, hydroxyethyl cellulose, hydroxypropyl cellulose, methyl vinyl ether-maleic anhydride copolymers, any salts thereof, any copolymers thereof, and mixtures thereof.

12. The catheter assembly (10) of claim 1, wherein the degradable coating (52, 54) comprises at least one member selected from the group consisting of polyethylene glycol, polyethylene oxide, carbohydrates, monosaccharides, oligosaccharides, polysaccharides, polyols, sugar alcohols, copolymers thereof and mixtures thereof.

## Patentansprüche

1. Eine Kathetervorrichtung (10) umfassend:
ein ausdehnbares Ballonelement (20) welches eine innere Oberfläche und eine äußere Oberfläche aufweist, angeordnet auf dem ausdehnbaren Ballonelement;
ein ausdehnbares intraluminales Medizinprodukt (40), welches eine innere Oberfläche und eine äußere Oberfläche aufweist, wobei diese auf dem ausdehnbaren Ballonelement angeordnet sind; und
eine abbaubare Beschichtung (50) in Kontakt mit wenigstens einem Teil der inneren Oberfläche oder äußeren Oberfläche des ausdehnbaren intraluminalen Medizinproduktes (40) und in Kontakt mit mindestens einem Teil der äußeren Oberfläche des ausdehnbaren Ballonelementes,
wobei die abbaubare Beschichtung eine Lage-für-Lage-Beschichtung (52, 54) ist, so ausgewählt, um das ausdehnbare intraluminale Medizinprodukt von dem ausdehnbaren Ballonelement unter Ausdehnung oder Zusammenziehung des ausdehnbaren Ballonelementes von einem aufgeblasenen Zustand unter Exponierung an eine Umgebung innerhalb des Körpers freizusetzen, wobei die Lage-für-Lage-Beschichtung wenigstens eine erste Schicht und wenigstens eine zweite Schicht umfasst, wobei die wenigstens eine erste Schicht ein Material umfasst welches eine negative Ladung trägt oder ein Material, welches eine positive Ladung trägt, und wobei jene wenigstens eine zweite Schicht ein Material umfasst, welches die entgegengesetzte Ladung zu der Ladung der ersten Schicht trägt.

2. Kathetervorrichtung (10) gemäß Patentanspruch 1, wobei die erste Schicht (52) oder die zweite Schicht (54) auf wenigstens einem Teil der äußeren Oberfläche des ausdehnbaren Ballonelementes angeordnet ist.

3. Kathetervorrichtung (10) gemäß Patentanspruch 2, wobei die erste Schicht (52) auf wenigstens einem Teil der äußeren Oberfläche des ausdehnbaren Ballonelementes (20) angeordnet ist, und die zweite Schicht (54) auf wenigstens einem Teil der inneren Oberfläche des ausdehnbaren Medizinproduktes angeordnet ist.

4. Kathetervorrichtung (10) gemäß Patentanspruch 2, wobei die erste Schicht (52) auf wenigstens einem Teil der äußeren Oberfläche des ausdehnbaren Ballonelementes (20) angeordnet ist, und die zweite Schicht (54) auf wenigstens einem Teil der äußeren Oberfläche des ausdehnbaren Medizinproduktes (40) angeordnet ist.

5. Kathetervorrichtung (10) gemäß Patentanspruch 1, wobei die wenigstens eine erste Schicht (52) und die wenigstens eine zweite Schicht (54) jeweils einen Vertreter umfasst, der ausgewählt ist aus der Gruppe bestehend aus Polyelektrolyten, Polyelektrolytkomplexen, anorganischen Partikeln, anorganischen Polymeren, anorganischen Lipiden, ionischen Polymeren, Proteinen, DNA und Mischungen daraus.

6. Kathetervorrichtung (10) gemäß Patentanspruch 1, wobei die wenigstens eine erste Schicht (52) und die wenigstens eine zweite Schicht (54) ein ionisches Polymer umfasst, ausgewählt aus der Gruppe bestehend aus Carboxyl-funktionalisierten Polymeren, Sulfat-funktionalisierten Polymeren, Amin-funktionalisierten Polymeren, Kohlehydraten, Monosachariden, Oligosachariden, Polysachariden, Polyolen, Zuckeralkoholen und Mischungen davon.

7. Kathetervorrichtung (10) gemäß Patentanspruch 6, wobei die wenigstens eine erste Schicht (52) und die wenigstens eine zweite Schicht (54) ein ionisches Polymer umfasst, ausgewählt aus der Gruppe bestehend aus Polyacrylsäure, Polymethacrylsäure, Polyethylenamin, Polysachariden, Alginsäure, Pektinsäure, Carboxymethylzellulose, Hyaluronsäure, Heparin, Chitosan, Carboxymethylchitosan, Carboxymethylstärke, Carboxymethyldextran, Heparinsulfat, Chondroitinsulfat, kationischem Guar, kationischer Stärke, Alginsäure, Pektinsäure, Hyaluronsäure, Salzen davon und Mischungen davon.

8. Kathetervorrichtung (10) gemäß Patentanspruch 1, wobei die erste Schicht (52) Heparin und die zweite Schicht (54) Chitosan umfasst.

9. Kathetervorrichtung (10) gemäß Patentanspruch 1, wobei die abbaubare Beschichtung (52, 54) so ausgewählt ist, dass sie das Medizinprodukt (40) von dem ausdehnbaren Ballonelement (20) in eine wasserhaltige Umgebung freisetzt.

10. Kathetervorrichtung (10) gemäß Patentanspruch 9, wobei die abbaubare Beschichtung (52, 54) ein Material umfasst, welches so ausgewählt ist, dass es sich in einer wasserhaltigen Umgebung auflöst.

11. Kathetervorrichtung (10) gemäß Patentanspruch 1, wobei die abbaubare Beschichtung (52, 54) wenigstens einen Vertreter umfasst, ausgewählt aus der Gruppe bestehend aus Polyethylenglykol, modifizierten Polyethylenglykolen, Polyethylenoxid, Blockcopolymere von Polyethylenoxid und Polypropylenoxid, Polysachariden, modifizierten Polysachariden, hydrophilem Polyurethan, hydrophilen Polyamiden, Hydroximethylmethacrylat (HEMA), Polyacrylsäure, Polyvinylalkohol, Polyvinylacetat, Polyvinylpyrrolidon, Cellulose, Carboxymethylcellulose, Methylcellulose, Hydroxyethylcellulose, Hydroxypropylcellulose, Methyl-Vinyl-Ethermaleinanhydrid-Copolymeren, einem Salz davon, einem Copolymer davon und Mischungen davon.

12. Kathetervorrichtung (10) gemäß Patentanspruch 1, wobei die abbaubare Beschichtung (52, 54) wenigstens einen Vertreter umfasst, ausgewählt aus der Gruppe bestehend aus Polyethylenglykol, Polyethylenoxid, Kohlehydraten, Monosachariden, Oligosachariden, Polysachariden, Polyolen, Zuckeralkohole, Copolymeren davon und Mischungen davon.

## Revendications

1. Ensemble formant cathéter (10), comprenant :
un élément (20) formant ballonnet dilatable, ayant une surface intérieure et une surface extérieure disposées sur l'élément formant ballonnet dilatable ;
un dispositif médical intraluminal dilatable (40) ayant une surface intérieure et une surface extérieure, et disposé sur l'élément formant ballonnet dilatable ; et
un revêtement dégradable (50), en contact avec au moins une partie de la surface intérieure ou de la surface extérieure du dispositif médical intraluminal dilatable (40) et en contact avec au moins une partie de la surface extérieure de l'élément formant ballonnet dilatable,
le revêtement dégradable étant un revêtement couche par couche (52, 54), choisi de façon à permettre au dispositif médical intraluminal dilatable de se détacher de l'élément formant ballonnet dilatable après dilatation ou contraction de l'élément formant ballonnet dilatable, à partir d'un état gonflé après exposition à un environnement à l'intérieur du corps, le revêtement couche par couche comprenant au moins une première couche (52) et au moins une deuxième couche (54), ladite au moins première couche comprenant un matériau portant une charge négative ou un matériau portant une charge positive, et ladite au moins une deuxième couche comprenant un matériau portant la charge opposée à celle de la première couche.

2. Ensemble formant cathéter (10) selon la revendication 1, dans lequel ladite première couche (52) ou ladite deuxième couche (54) est disposée sur au moins une partie de ladite surface extérieure dudit élément formant ballonnet dilatable.

3. Ensemble formant cathéter (10) selon la revendication 2, dans lequel ladite première couche (52) est disposée sur au moins une partie de ladite surface extérieure dudit élément formant ballonnet dilatable (20), et ladite deuxième couche (54) est disposée sur au moins une partie de ladite surface intérieure dudit dispositif médical dilatable.

4. Ensemble formant cathéter (10) selon la revendication 2, dans lequel ladite première couche (52) est disposée sur au moins une partie de ladite surface extérieure dudit élément formant ballonnet dilatable (20), et ladite deuxième couche (54) est disposée sur au moins une partie de ladite surface extérieure dudit dispositif médical dilatable (40).

5. Ensemble formant cathéter (10) selon la revendication 1, dans lequel ladite au moins première couche (52) et ladite au moins une deuxième couche (54) comprennent chacun un élément choisi dans le groupe consistant en les polyélectrolytes, les complexes polyélectrolytiques, les particules inorganiques, les polymères inorganiques, les lipides inorganiques, les polymères ioniques, les protéines, l'ADN et les mélanges de ceux-ci.

6. Ensemble formant cathéter (10) selon la revendication 1, dans lequel ladite au moins une première couche (52) et ladite au moins une deuxième couche (54) comprennent un polymère ionique choisi dans le groupe consistant en les polymères à fonctionnalité carboxylique, les polymères à fonctionnalité sulfate, les polymères à fonctionnalité amine, les hydrates de carbone, les monosaccharides, les oligosaccharides, les polysaccharides, les polyols, les alcools de sucre et les mélanges de ceux-ci.

7. Ensemble formant cathéter (10) selon la revendication 6, dans lequel ladite au moins une première couche (52) et ladite au moins une deuxième couche (54) comprennent un polymère ionique choisi dans le groupe comprenant le poly(acide acrylique), le poly(acide méthacrylique), la polyéthylèneamine, les polysaccharides, l'acide alginique, l'acide pectique, la carboxyméthyl-cellulose, l'acide hyaluronique, l'héparine, le chitosane, le carboxyméthylchitosane, le carboxyméthylamidon, le carboxyméthyldextrane, le sulfate d'héparine, le sulfate de chondroïtine, la gomme de guar cationique, l'amidon cationique, l'acide alginique, l'acide pectique, l'acide hyaluronique, l'un quelconque des sels de ceux-ci, et les mélanges de ceux-ci.

8. Ensemble formant cathéter (10) selon la revendication 1, dans lequel ladite première couche (52) comprend de l'héparine et ladite deuxième couche (54) comprend du chitosane.

9. Ensemble formant cathéter (10) selon la revendication 1, dans lequel le revêtement dégradable (52, 54) est choisi de façon à permettre au dispositif médical (40) de se détacher de l'élément formant ballonnet dilatable (20) dans un environnement à base aqueuse.

10. Ensemble formant cathéter (10) selon la revendication 9, dans lequel le revêtement dégradable (52, 54) comprend un matériau qui est choisi de façon à se dissoudre dans un environnement à base aqueuse.

11. Ensemble formant cathéter (10) selon la revendication 1, dans lequel le revêtement dégradable (52, 54) comprend au moins un élément choisi dans le groupe consistant en le polyéthylèneglycol, les polyéthylèneglycols modifiés, le poly(oxyde d'éthylène), les copolymères à blocs de poly(oxyde d'éthylène) et de poly(oxyde de propylène), les polysaccharides, les polysaccharides modifiés, les polyuréthannes hydrophiles, les polyamides hydrophiles, le méthacrylate d'hydroxyéthyle (HEMA), le poly(acide acrylique), le poly(alcool vinylique), le poly(acétate de vinyle), la polyvinylpyrrolidone, la cellulose, la carboxyméthyl-cellulose, la méthylcellulose, l'hydroxyéthylcellulose, l'hydroxypropylcellulose, les copolymères méthylvinyléther-anhydride maléique, l'un quelconque des sels de ceux-ci, l'un quelconque des copolymères de ceux-ci, et les mélanges de ceux-ci.

12. Ensemble formant cathéter (10) selon la revendication 1, dans lequel le revêtement dégradable (52, 54) comprend au moins un élément choisi dans le groupe consistant en le polyéthylèneglycol, le poly(oxyde d'éthylène), les hydrates de carbone, les monosaccharides, les oligosaccharides, les polysaccharides, les polyols, les alcools de sucre, les copolymères de ceux-ci et les mélanges de ceux-ci.
